(19) Europäisches Patentamt / European Patent Office / Office européen des brevets

(11) EP 3 352 293 A1

(12) **EUROPEAN PATENT APPLICATION**

(43) Date of publication:
**25.07.2018 Bulletin 2018/30**

(51) Int Cl.:
***H01M 12/08*** *(2006.01)* *H01M 10/36* *(2010.01)*

(21) Application number: **17157540.0**

(22) Date of filing: **23.02.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **20.01.2017 US 201762448480 P**

(71) Applicant: **Albemarle Corporation**
**Baton Rouge, LA 70801 (US)**

(72) Inventors:
- **GE, Zhongxin**
  **Baton Rouge, LA 70810 (US)**
- **MANMARAN, Thanikavelu**
  **Baton Rouge, LA 70810 (US)**
- **O'DAY, Joseph**
  **Baton Rouge, LA 70809 (US)**

(74) Representative: **Uexküll & Stolberg Partnerschaft von Patent- und Rechtsanwälten mbB**
**Beselerstraße 4**
**22607 Hamburg (DE)**

# (54) QUATERNARY AMMONIUM HALIDES WITH ETHER FUNCTIONAL GROUPS FOR USE AS BATTERY ELECTROLYTES

(57) An electrolyte solution and a flow cell battery are included herein. The electrolyte solution generally includes a electrolyte solution including one or more class A quat halides. The flow cell battery includes an electrolyte solution including one or more class A quat halides.

EP 3 352 293 A1

## Description

### TECHNICAL FIELD

In general, the present disclosure relates to electrolyte solutions and flow cell batteries including an electrolyte solution.

### BACKGROUND

The reduction of diatomic bromine to two bromide ions has been used in the electrolyte fluids of flowable batteries for decades. Battery types whose function depends on the bromine half reaction include zinc bromide, hydrogen bromide and vanadium bromide batteries. Such batteries generally contain a flowing electrolyte, which allows for increased electrolyte volume, extending the life of the battery before recharge is required. It also enables the power of the battery to be recharged by replacement of spent electrolyte fluids, while allowing for ordinary recharge methods to be used, if desired.

### BRIEF SUMMARY

However, even at relatively low concentrations, diatomic bromine has a propensity to form a vapor phase which separates out of the liquid electrolyte, interfering with the recharge of bromine-containing batteries. For this reason, it is necessary to keep the free diatomic bromine concentration in the electrolyte low enough such that vapor phase formation does not occur. Furthermore, aside from the tendency of free bromine to vaporize, an abundance of free bromine in solution can lead to the direct oxidation of metal electrodes, such as the zinc electrode in the case of zinc bromide batteries. Thus, while bromine must be solvated in order to function as an electron donor/acceptor during battery use/recharge, the concentration is optimally maintained at only a low level.

In order to meet these requirements and yet have appreciable battery life, the electrolyte solution of bromine-containing flow batteries can contain an agent which complexes with elemental bromine, preventing the formation of a bromine vapor phase. The complex can form a separate layer from the rest of the electrolyte, essentially sequestering the complexed bromine away from the electrode in an oily phase. The degree to which the oily phase forms depends, to some extent, upon the identity of the complexing agent. The complexing agent releasably retains diatomic bromine, acting as a reservoir by releasing additional diatomic bromine into the electrolyte solution as that present in the solution is reduced to bromide ions. During battery cell recharging, as the bromine is released, the complexing agent, which is soluble in the electrolyte, is regenerated and again becomes a component of the circulating electrolyte solution.

Quaternary nitrogen halide-based complexing agents are generally able to complex with at least one, and in some cases, four or more diatomic bromine molecules. In theory, the more diatomic bromine with which a complexing agent is able to complex, the longer the agent will be able to release bromine and the longer the life of the battery cell before recharge is needed.

It has now been observed that increasing aliphatic chain length of quaternary halide compound substituents improves the compound's ability to complex with diatomic bromine, giving complexes with increased amounts of sequestered bromine per molecule of quaternary compound. However, in order to complex diatomic bromine, the molecule must also be soluble in an aqueous electrolyte solution; the bromine-complexing ability is not extant unless the complexing agent is solvated. With respect to quaternary halide compounds, attempts to increase bromine complexing ability by increasing the aliphatic chain length of nitrogen substituents has been met with the practical limitation of decreasing solubilities. Thus, molecules which have been used as complexing agents are generally relatively simple quaternary ammonium compounds bearing short aliphatic substituents, such as methyl and ethyl groups, in order to avoid impeding electrolyte solubility.

Flow-battery cells may be put to a wide variety of ultimate uses. Such uses span a range of temperatures. The solubility of quaternary ammonium halide complexing agents ("quats") can be heavily temperature-dependent, with quaternary ammonium halide compounds used heretofore, such as dimethylethylpropyl ammonium bromide (DMEP) having a cloud point of about 23.2°C (where "cloud point" as used herein is that of a solution which is 0.7M in DMEP and 2.5M in $ZnBr_2$). Many complexing agents of high sequestering efficiency have cloud points which are above about 20°C, and thus cannot be relied upon to remain solvated in aqueous electrolyte solutions at temperatures below their respective cloud points, further limiting the uses to which the batteries can be put. "High sequestering efficiency" is defined, for purposes herein as leaving less than 1.5 wt% free bromine from an initial mixture which is 0.7 M in complexing agent, 0.5 M in zinc bromide, and 2 M bromine."

It has been found that the use of specific types of ether-containing quats, designated as "class A" quats which are otherwise of high solubility (characterized by a low cloud point) with other quats can give a mixture having a solubility which is increased with respect to the other quat by itself, and most surprisingly, a free bromine characteristic which is

surprisingly low in comparison to the free bromine characteristics of the individual mixture components by themselves. By "cloud point" is meant the cloud point of an aqueous solution containing 0.7 M complexing agent and 2.5 M zinc bromide.

Thus, in one aspect, the present disclosure provides an electrolyte and a bromine-containing flow cell battery comprising the electrolyte. The electrolyte comprises one or more class A quats each having a molecular structure selected from the group consisting of:

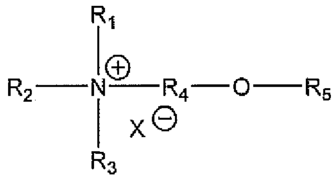

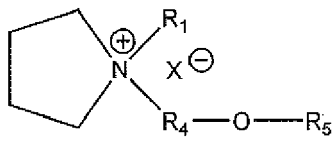

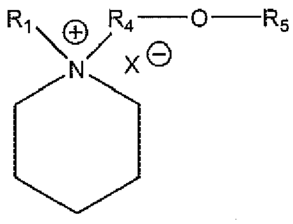

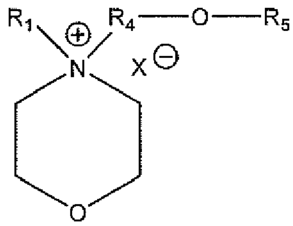

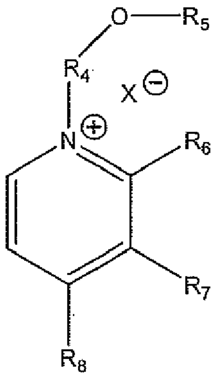

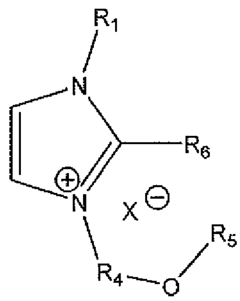

wherein $X^-$ is $Br^-$ or $Cl^-$, or the quat is a mixture of both bromides and chlorides; $R_1$, $R_2$, $R_3$, and $R_5$ are, independently, alkyl substituents having 12 or fewer carbon atoms; where $R_1$, $R_2$ and $R_3$, can be hydrogen. $R_4$ is an alkyl chain having in the range of about 1 to about 10 carbon atoms. $R_5$ is the terminating alkyl group (to the right of the ether oxygen in all structures), $R_4$ is the bridging group (to the left of the ether oxygen in all structures), $R_1$, and, if needed, $R_2$ and $R_3$, are the remaining groups attached to the quaternary nitrogen. Other R groups, such as those ring substituents or those attached to non-quaternary nitrogen atoms, are labeled $R_6$, $R_7$, etc., and are, independently, hydrogen or alkyl substituents having 12 or fewer carbon atoms, or, in other aspects, from about 1 to about 7 carbon atoms, or from about 1 or 2 to about 4 carbon atoms.

Furthermore, it has been found that mixtures of class A quats with specific quats designated as "class B quats" can have an acceptable cloud point and a surprisingly low free bromine. Thus, in an additional aspect, the electrolyte additionally comprises one or more tetra-alkyl quats of the following structure:

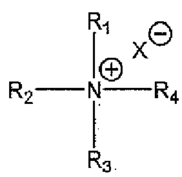

or one or more quats of the following structures:

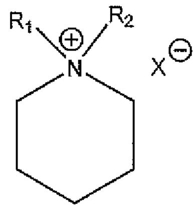
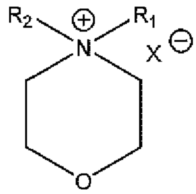
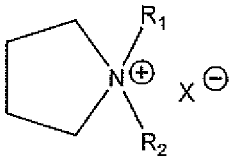
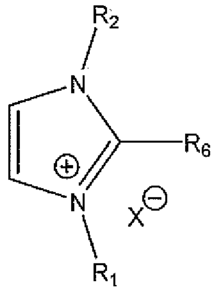
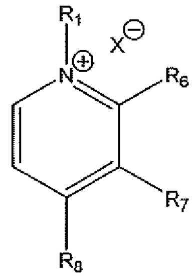

wherein $X^-$ is $Br^-$ or $Cl^-$, or the quat is a mixture of both bromides and chlorides; $R_1$, $R_2$, $R_3$, are, independently, hydrogen or alkyl substituents having 12 or fewer carbon atoms; $R_4$ is an alkyl chain having in the range of about 1 to about 10 carbon atoms. Other R groups, such as those ring substituents or those attached to non-quaternary nitrogen atoms, are labeled $R_6$, $R_7$, etc., and are, independently, hydrogen or alkyl substituents having 12 or fewer carbon atoms, or, in other aspects, from about 1 to about 7 carbon atoms, or from about 1 or 2 to about 4 carbon atoms.

In further aspects, the one or more class B quats have the following structure:

$$C\text{-}(CH_2)n\text{-}D$$

wherein C and D each are each independently selected from the group consisting of:

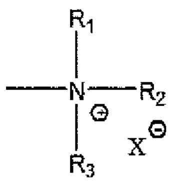
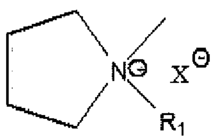
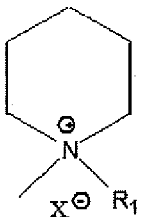
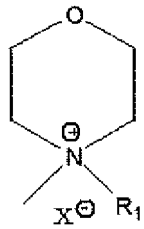
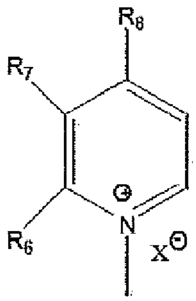
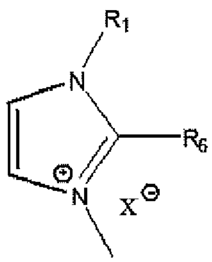

wherein $X^-$ is Br- or Cl-, or the quat is a mixture of both bromides and chlorides, n is in the range of 1 to 100, $R_1$, $R_2$, $R_3$, are, independently, hydrogen or alkyl substituents having about 12 or fewer carbon atoms, and $R_6$, $R_7$ and $R_8$ are, independently, hydrogen or alkyl substituents having 12 or fewer carbon atoms. In further aspects, the one or more class B quats is/are dimethylethyl gemini quat bromide with butyl linkage having the following structure:

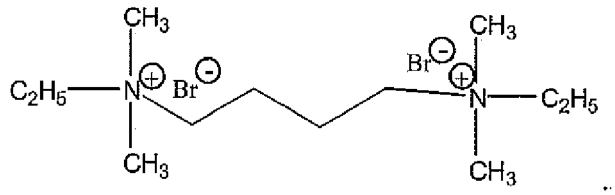

In one or more aspects, the electrolyte solution includes one or more ether-containing class A quat halides, each having the following molecular structure.

$$A-[(CH_2)_E-O-(CH_2)_F]_n-B$$

wherein A and B each are each independently selected from the group consisting of:

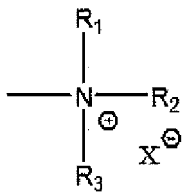

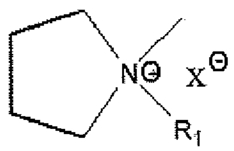

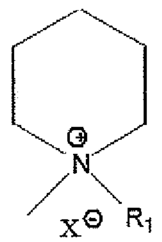

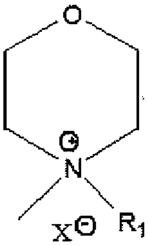

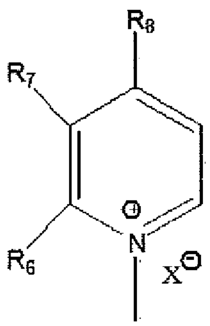

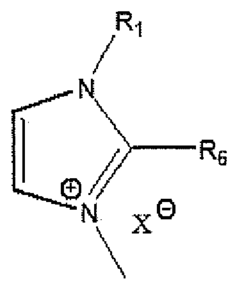

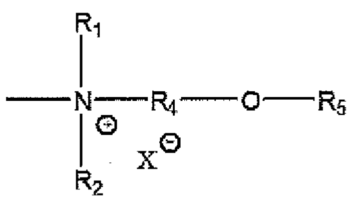

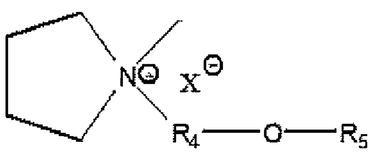

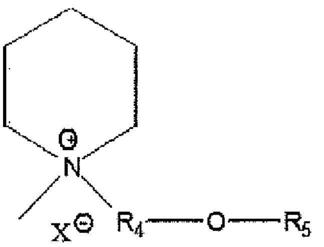

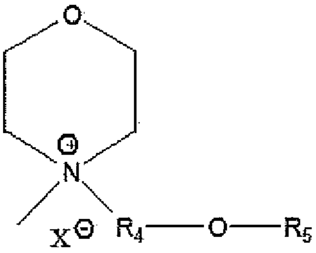

wherein E and F are each independently in the range of 1 to 10, n is in the range of 1 to 100, $X^-$ is $Br^-$ or $Cl^-$, or the quat is a mixture of both bromides and chlorides, $R_1$, $R_2$, $R_3$, are, independently, hydrogen or alkyl substituents having about 12 or fewer carbon atoms, $R_4$ is an alkyl chain having in the range of about 1 to about 10 carbon atoms, $R_5$ is an alkyl group having in the range of about 1 to about 6 carbon atoms, and, $R_6$, $R_7$ and $R_8$ are, independently, hydrogen or alkyl substituents having 12 or fewer carbon atoms. In one or more aspects, $R_1$, $R_2$ and $R_3$ are, independently, hydrogen or alkyl substituents having in the range of about 1 and about 7 carbon atoms. In further aspects, $R_4$ is an alkyl chain having in the range of about 1 to about 4 carbon atoms. In one or more aspects, $R_5$ is an alkyl group having in the range of about 1 to about 4 carbon atoms. In still further aspects, the one or more class A quats is/are dimethylethyl gemini quat bromide with diethylether linkage having the following structure:

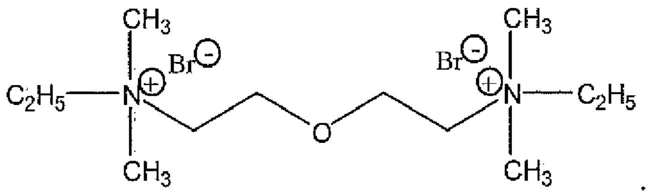

While multiple embodiments are disclosed, still other embodiments will become apparent to those skilled in the art from the following detailed description. As will be apparent, certain embodiments, as disclosed herein, are capable of modifications in various obvious aspects, all without departing from the spirit and scope of the claims as presented herein. Accordingly, the detailed description is to be regarded as illustrative in nature and not restrictive.

## DESCRIPTION OF EMBODIMENTS

Types of flow batteries in which the electrolytes of the present disclosure can be used include, but are not limited to, Zinc bromide-type flow cell batteries, Vanadium bromide-type flow cell batteries, Polysulfide bromine-type flow cell batteries and Hydrogen bromine flow cell batteries.

### *Class A Quat*

In one aspect, the present disclosure provides a zinc bromide electrolyte solution comprising at least one ether-containing "class A" quat. One class A quat has the following structure:

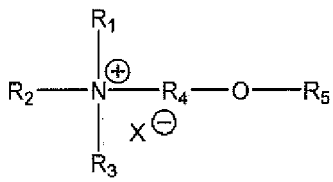

wherein $X^-$ is $Br^-$ or $Cl^-$, or the quat is a mixture of both bromides and chlorides; $R_1$, $R_2$ and $R_3$ are, independently, hydrogen or alkyl substituents having 12 or fewer carbon atoms, or more preferably between about 1 and about 7 carbon atoms. $R_4$ is an alkyl chain having in the range of 1 to 10, or more preferably in the range of from about 1 to about 4, carbon atoms. $R_5$ is an alkyl group having in the range of 1 to 10, or more preferably in the range of from about 1 to about 4 carbon atoms. In one aspect, $R_1$, $R_2$ and $R_3$ are, independently, ethyl or methyl, and $R_4$ is ethyl or methyl and $R_5$ is methyl or ethyl. In one aspect, the sum of the lengths of $R_4$, $R_5$ and the interconnecting ether oxygen is in the range of about 3 to about 12 atoms, or in other aspects, in the range of about 4 to about 6 atoms. In yet another aspect, the

sum of the foregoing lengths is 4. In yet another aspect, $X^-$ is $Br^-$.

In still another aspect of the present disclosure, the electrolyte solution comprises two class A quat halides, a first quat and a second quat. In a further aspect, the both class A quats halides are trialkyl, ether quat halides, wherein the nitrogen bears three alkyl groups as well as an alkyl group between the nitrogen and the ether oxygen comprising in the range of about 1 to about 6 carbon atoms, and wherein the ether oxygen is also connected to another alkyl group having 1, 2, 3, 4, 5 or 6 carbons. In further aspects, the first class A quat halide is (2-methoxyethyl)-triethylammonium bromide, a triethylether quat having the following structure:

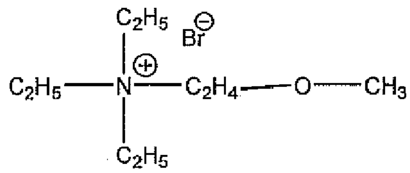

and the second class A quat halide is diethylmethyl-(2-methoxyethyl)ammonium bromide, a diethylmethylether quat having the following structure:

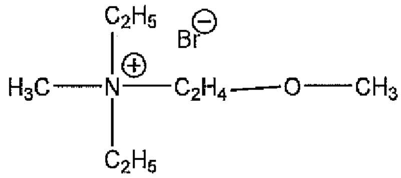

Other class A quat halides include ether-containing quats having a molecular structures selected from the group consisting of the following structures:

I

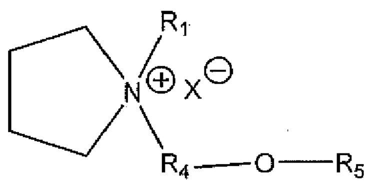

II

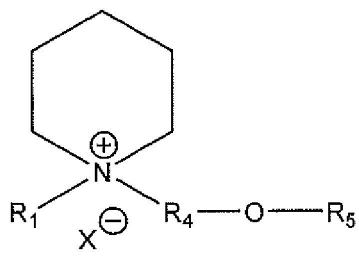

III

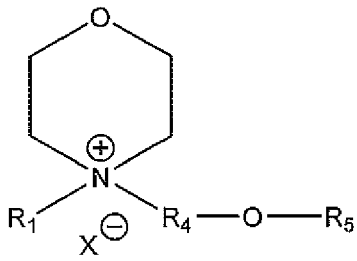

IV

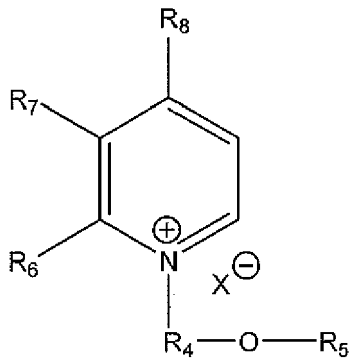

V

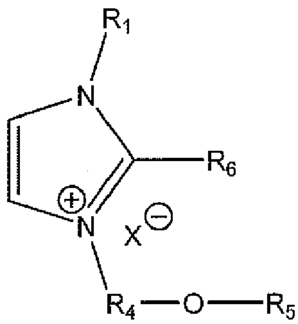

VI

wherein $X^-$ is $Br^-$ or $Cl^-$, or the quat is a mixture of both bromides and chlorides; $R_1$, $R_2$, $R_3$ are, independently, hydrogen or alkyl substituents having 12 or fewer carbon atoms, or, in other aspects, from about 1 to about 7 carbon atoms, or from about 1 or 2 to about 4 carbon atoms. $R_4$ is an alkyl chain having in the range of 1 to 10, or, in other aspects, in the range of from about 1 or about 2 to about 4, carbon atoms. $R_5$ is an alkyl group having in the range of 1 to 6, or, in other aspects, in the range of from about 1 or 2 to about 4 carbon atoms. In one aspect, $R_1$, $R_2$ and $R_3$ are, independently, ethyl or methyl, and $R_4$ is ethyl or methyl and $R_5$ is methyl or ethyl. In one aspect, the sum of the lengths of $R_4$, $R_5$ and the interconnecting ether oxygen is in the range of about 3 to about 12 atoms, or in other aspects, in the range of about 4 to about 6 atoms. In yet another aspect, the sum of the foregoing lengths is 4. Other R groups, such as those ring substituents or those attached to non-quaternary nitrogen atoms, are labeled $R_6$, $R_7$, etc., and are, independently, hydrogen or alkyl substituents having 12 or fewer carbon atoms, or, in other aspects, from about 1 to about 7 carbon atoms, or from about 1 or 2 to about 4 carbon atoms.

In further embodiments, the present disclosure provides an electrolyte solution, e.g., a zinc bromide electrolyte solution, comprising one or more ether-containing class A quats, each quat having the following structure:

$$A—[(CH_2)_E\text{-}O\text{-}(CH_2)_F]_n—B$$

wherein A and B are each independently selected from the group consisting of:

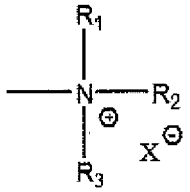
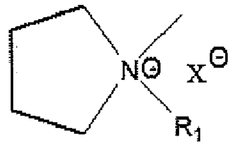

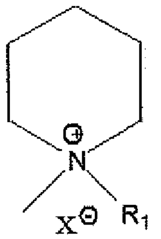
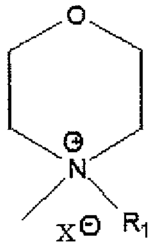

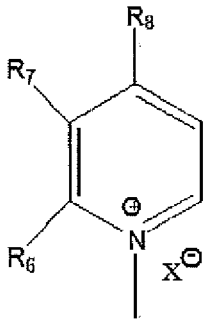
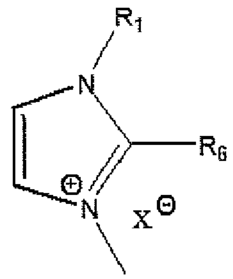

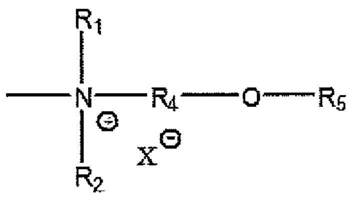
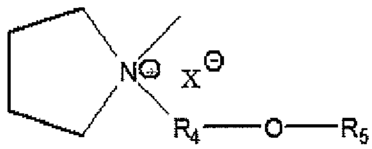

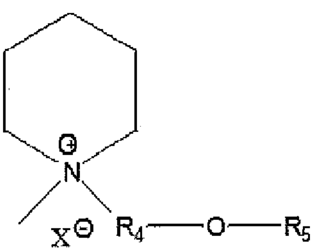
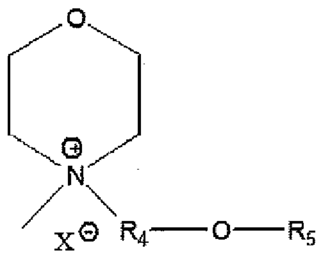

wherein $X^-$ is Br- or Cl-, or the quat is a mixture of both bromides and chlorides, and wherein E and F are each 10 or less, or preferably are each in the range of 1 to 10, or more preferably E and F are each in the range of 1 to 3. In some embodiments, E may be 1, 2, 3, 4, 5, 6, 7, 8, 9 or 10, and F may be 1, 2, 3, 4, 5, 6, 7, 8, 9 or 10. In some embodiments, n is 100 or less, or preferably in the range of 1 to 100, or more preferably in the range of 1 to 10. In some embodiments n may be 1, 2, 3, 4, 5, 6, 7, 8, 9 or 10. $R_1$, $R_2$ and $R_3$ are, independently, hydrogen or alkyl substituents having 12 or fewer carbon atoms, or more preferably between about 1 and about 7 carbon atoms. $R_4$ is an alkyl chain having in the range of 1 to 10 carbon atoms, or more preferably in the range of from about 1 to about 4 carbon atoms. $R_5$ is an alkyl group having in the range of 1 to 10 carbon atoms, or more preferably in the range of from about 1 to about 4 carbon atoms. In some embodiments, $R_1$, $R_2$ and $R_3$ are, independently, ethyl or methyl, and $R_4$ is ethyl or methyl and $R_5$ is

methyl or ethyl. In some embodiments, the sum of the lengths of $R_4$, $R_5$ and the interconnecting ether oxygen is in the range of about 3 to about 12 atoms, or in other aspects, in the range of about 4 to about 6 atoms. In yet another aspect, the sum of the foregoing lengths is 4. $R_6$, $R_7$, and $R_8$ are, independently, hydrogen or alkyl substituents having 12 or fewer carbon atoms, or, in other aspects, from about 1 to about 7 carbon atoms, or from about 1 or 2 to about 4 carbon atoms. In some embodiments, the one or more class A quats is/are dimethylethyl gemini quat bromide with diethylether linkage having the following structure:

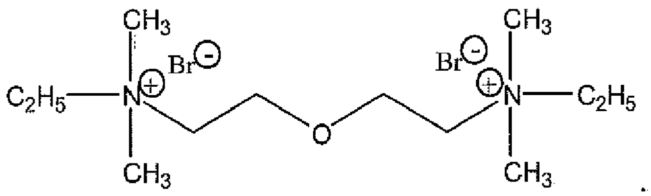

*Class B Quat*

In further aspects, the present disclosure provides a zinc bromide electrolyte solution comprising one or more class A quat halides and one or more "class B" quat halides. Class B quat halides can be of a number of types. In one aspect, the class B quat halide can be one or more tetra-alkyl quats comprising four alkyl substituents $R_1$, $R_2$, $R_3$ and $R_4$, wherein the substituents are independently alkyl groups comprising in the range of from about 1 to about 10 carbon atoms, and in other aspects, in the range of about 1 to about 6, or about 1 to about 4 carbon atoms. In one aspect, the class B quat halide is triethylpropylammonium bromide:

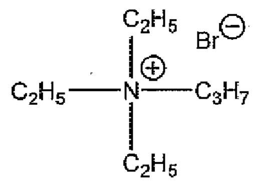

In another aspect, the class B quat is an alkylpiperidinyl quat halide, wherein the piperidine ring may be alkyl substituted, and the quaternary nitrogen can bear, in addition to the piperidinyl linkages, one or two alkyl groups. In one aspect, the piperidinyl ring is unsubstituted

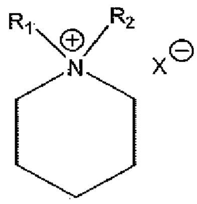

In some aspects, the alkyl groups, $R_1$, $R_2$, are, independently, hydrogen or alkyl substituents having 12 or fewer carbon atoms, or more preferably between about 1 and about 7 carbon atoms. In one particular aspect of the invention, $R_1$, and $R_2$ are, independently, ethyl, methyl or propyl. In another particular aspect, the piperidinyl ring is unsubstituted and $R_1$ and $R_2$ are ethyl groups and $X^-$ is $Br^-$.

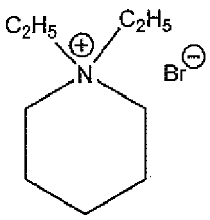

In another aspect, one of the alkyl groups is a propyl group.

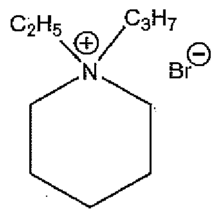

In further aspects, the class B quat halide comprises one or more quaternary compounds having a molecular structure selected from the group consisting of the following structures:

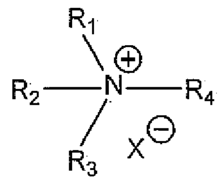

I

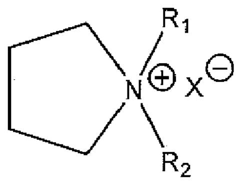

II

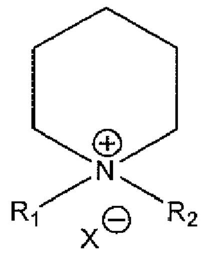

III

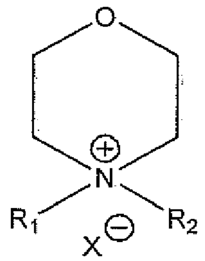

IV

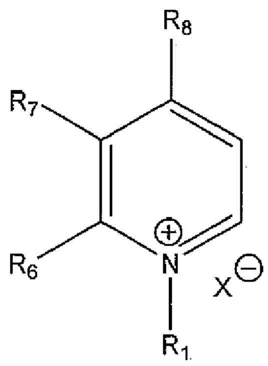

V

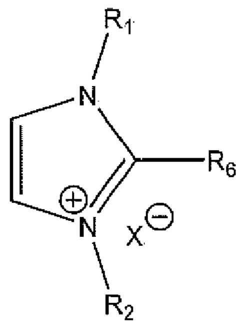

VI

wherein $X^-$ is $Br^-$ or $Cl^-$, or the quat is a mixture of both bromides and chlorides; $R_1$, $R_2$, $R_3$ are, independently, hydrogen or alkyl substituents having 12 or fewer carbon atoms, or, in other aspects, from about 1 to about 7 carbon atoms, or from about 1 or 2 to about 4 carbon atoms. $R_4$ is an alkyl chain having in the range of 1 to 10, or, in other aspects, in the range of from 1 or 2 to 4, carbon atoms. In further aspects, $R_1$, $R_2$, $R_3$ and $R_4$ are, independently, ethyl or methyl. Other R groups, such as those ring substituents or those attached to non-quaternary nitrogen atoms, are labeled $R_6$, $R_7$, etc., and are, independently, hydrogen or alkyl substituents having 12 or fewer carbon atoms, or, in other aspects, from about 1 to about 7 carbon atoms, or from about 1 or 2 to about 4 carbon atoms.

In further embodiments, the class B quat halide comprises one or more quaternary compounds having the following structure:

$C\text{-}(CH_2)n\text{-}D$

wherein C and D are each independently selected from the group consisting of:

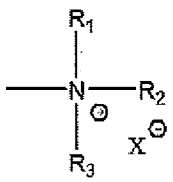

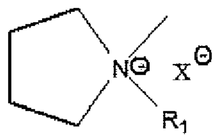

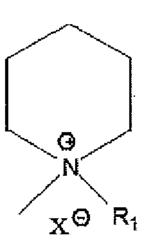

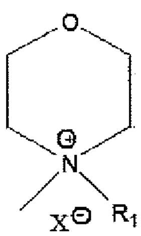

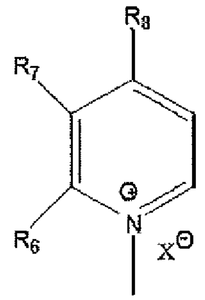 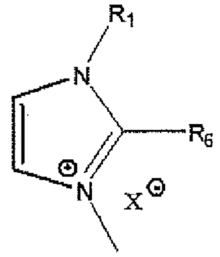

wherein $X^-$ is Br- or Cl-, or the quat is a mixture of both bromides and chlorides, and wherein n is 100 or less, or preferably in the range of 1 to 100, or more preferably in the range of 1 to 10. In some embodiments n may be 1, 2, 3, 4, 5, 6, 7, 8, 9 or 10. $R_1$, $R_2$ and $R_3$ are, independently, hydrogen or alkyl substituents having 12 or fewer carbon atoms, or more preferably between about 1 and about 7 carbon atoms. $R_4$ is an alkyl chain having in the range of 1 to 10 carbon atoms, or more preferably in the range of from about 1 to about 4 carbon atoms. $R_5$ is an alkyl group having in the range of 1 to 10 carbon atoms, or more preferably in the range of from about 1 to about 4 carbon atoms. In some embodiments, $R_1$, $R_2$ and $R_3$ are, independently, ethyl or methyl. $R_6$, $R_7$, and $R_8$ are, independently, hydrogen or alkyl substituents having 12 or fewer carbon atoms, or, in other aspects, from about 1 to about 7 carbon atoms, or more preferably from about 1 or 2 to about 4 carbon atoms. In some embodiments, the one or more class B quats is/are dimethylethyl gemini quat bromide with butyl linkage having the following structure:

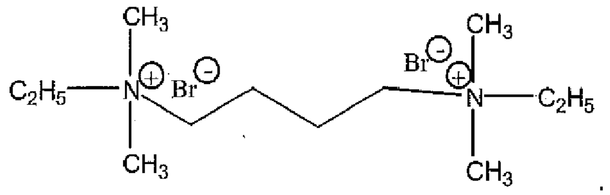

The electrolytes described in this present disclosure, both those that comprises the class A quat halide, and those that comprise a mixture of class A and class B quat halides are suitable for membraneless and membrane-containing aspects. In other aspects, the present disclosure provides a bromine-containing flow cell batteries containing the electrolyte solution.

In some embodiments, the present disclsoure provides a flow cell battery, such as a zinc bromide flow cell battery, comprising an electrolyte solution comprising one or more class A quat halides, or a mixture of one or more class A quat halides and one or more class B quat halides.

*Electrolyte Solution*

In one aspect, the complexing agent is a class A quat halide and is present in the electrolyte solution in a concentration in the range of about 0.1 to about 3.0 moles per liter, and in other aspects, in the range of about 0.2 to about 2.0 moles per liter, and in still other aspects, in the range of about 0.5 to about 1.0 moles per liter, based upon the total volume of the electrolyte solution.

In another aspect, the electrolyte solution comprises both at least one class A quat halide and at least one class B quat halide; wherein the molar ratio of class A to class B quat halide is in the range of from about 0.02 to about 50, in a narrower aspect, in the range of from about 0.2 to about 5, and in a further narrow aspect, about 1:1. The total molarity (or wt%, if more appropriate) of the class A and class B quats is in the range of from about 0.1M to about 3.0 M, and in a narrower aspect, in the range of from about 0.5M to about 1.0M.

The electrolyte solutions of the present disclosure can be used with a wide range of zinc bromide concentrations, including those of common use in the art. In one aspect of the present solution, the zinc bromide solution has a zinc bromide concentration in the range of from about 0.1 to about 3M. In a narrower aspect, the zinc bromide concentration is in the range of from about 1.5 to about 2.5M.

The electrolyte solution can be used in a wide variety of flow cell batteries, such as membrane-containing and membraneless designs known in the art. Necessary battery components include a flow cell with the bipolar electrodes and auxillary equipment such as pumps, electrolyte reservoir and a bromine complex storage. Other battery components which can be used with batteries containing the electrolyte solution include Bromine, Zinc Chloride, Ammonium Chloride and Potassium Chloride.

In general, the electrolyte solutions of the present disclosure have a plating efficiency in the range of about 50% to about 100% , and in other aspects, in the range of from 75% to about 100%.

The foregoing plating efficiency parameters are as follows:

Test Conditions: 25°C;

2-electrode cell set-up;

Working Electrode:

Conductive graphite rod (d=0.635cm or 1/4 in, length 5cm, surface area~10cm$^2$)

Counter/Ref Electrode:

Conductive graphite rod (d=0.635cm or 1/4 inch)

Equipment: MTI battery tester

Electrolyte: 35mL 2.5m $ZnBr_2$, 0.05M $Br_2$ and 0.7M Polybromide complex

30mA for 5 hours (total ~150mAh plating capacity) non-stirred condition

Working electrodes were thoroughly cleaned by DI water rinse and dried after zinc plating. Zinc plated on working electrode was determined by measuring weight difference of working electrode before and after plating test. Zinc plating efficiencies were calculated by real zinc weight over theoretical zinc weight, which was obtained by Faraday's law of electrolysis assuming 100% conversion from 180mAh capacity.

The class A quat-containing electrolyte solutions of the present disclosure generally have cloud points (as defined herein) at temperatures of less than about 25°C, in some aspects, less than about 0°C, and in still other aspects, less than about -10°C. The electrolyte solutions of the present disclosure comprising both class A and class B quats generally have cloud points of less than about 25°C, with some mixtures exhibiting cloud points of less than 5°C.

The forward operation of a bromine-containing battery generally involves the conversion of elemental bromine to ionic bromine. The quat/$Br_2$ complex results from an equilibrium reaction in which the $Br_2$ is released from the complex as the concentration of free $Br_2$ in the aqueous electrolyte drops during battery operation. In its fully charged state, the electrolyte solution inevitably contains some degree of uncomplexed bromine. A measure of a quat's ability to complex elemental bromine is the amount of elemental bromine left in solution when the complexation reaction proceeds to equilibrium. Such bromine is referred to as "free bromine." Measurement of free bromine in aqueous phase is set forth in Example 1.

The amount of free bromine depends upon characteristics of the complexing agent, such bromine-holding capacity, as well as the ease with which bromine disassociates from the complexing agent. In aspects in which the class A quat is used without the use of a class B quat, it is preferred that the free bromine of the quat, as measured by the procedure of Example 1, be less than about 1.5 wt%, and in narrower aspects, less than about 0.7wt%. If a class A/class B quat mixture is being used, it is preferred that the free bromine of the mixture be less than about 1.0 wt%, and in narrower aspects, less than about 0.5wt%.

The bromine-containing cells of the present disclosure can generally be operated at a wide range of temperatures. While other complexing agents in the art become crystalline at low temperatures, cells of the present disclosure can generally be operated at temperatures as low as 0°C, and in other aspects, as low as -10°C.

In general the quaternary ammonium bromide compounds disclosed herein can be used in the electrolyte solutions which include diatomic bromine as a component. Such flow batteries include, for example, zinc bromide, hydrogen bromide and vanadium bromide batteries. One aspect of the present disclosure is a zinc bromide battery comprising an electrolyte solution containing one or more class A quat halides, or a mixture of one or more class A quat halides with one or more class B quat halides. In additional aspects, the zinc bromide battery contains an electrolyte solution comprising one or more class A quat halides of the following structure:

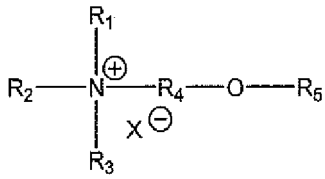

and optionally, one or more class B quat halides of the following types:

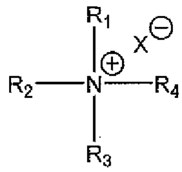

and

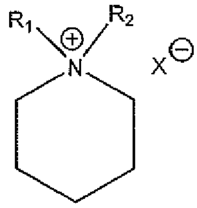

wherein the R-substituents are as given herein for the corresponding structures. In narrower aspects, the zinc bromide battery comprises an electrolyte solution comprising two class A quat halides of the following structures:

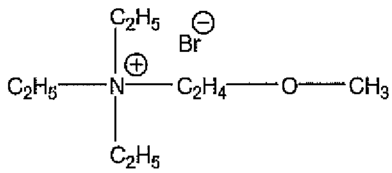

and

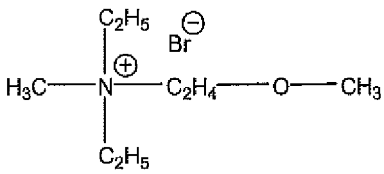

In another narrower aspect, the zinc electrolyte battery comprises a class A quat halide of the following structure:

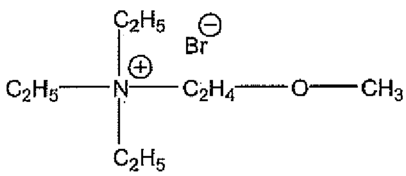

and class B quat halide of one of the following structures:

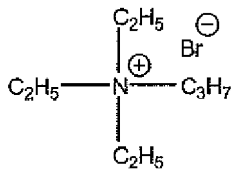

or

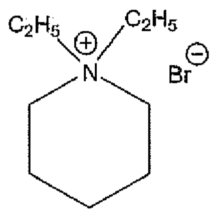

or

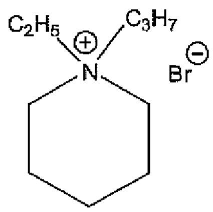

The complexing agents can be used in membraneless aspects. In one aspect, the present disclosure a membraneless flow cell battery comprising an electrolyte solution comprising one or more class A quat halides, or a mixture of one or more class A quat halides and one or more class B quat halides

**EXAMPLES**

**Example 1**

Measurement of free bromine in aqueous phase:

Two slightly different methods were used to prepare the electrolyte compositions, A) one containing 0.7M quat and B) the other containing 0.8M quat. In composition A, 2.0 moles of bromine was added to an aqueous solution containing 0.5 moles of zinc bromide and 0.7 moles of the quat. The two-phase mixture was stirred for 24 hrs. and then the phases were allowed to settle. The top aqueous phase was sampled for free bromine measurement. In composition B, 1.44 moles of bromine was added to an aqueous solution containing 0.5 moles of zinc bromide, 0.4 moles of zinc chloride and 0.8 moles of the quat. The top aqueous phase obtained after stirring for 24 hrs. was used for free bromine measurement.

A 250-ml Erlenmeyer flask was charged with 50 ml of deionized water and weighed (A). A sample of the clear aqueous phase was passed through glass wool to remove any suspended organic phase and then added to the Erlenmeyer flask. The flask was weighed again (B) and the difference in weight (B-A) was noted as the weight of the sample. About 20 ml of 20% potassium iodide solution followed by about 5 ml of starch solution was added to the flask. The resulting dark mixture was titrated against 0.02N sodium thiosulfate solution. The end-point was the disappearance of purple color. Free bromine (wt%) was calculated in the following manner:

Wt% free $Br_2$ = Volume (ml) of sodium Thiosulfate x Normality of Sodium thisosulfate/ Sample Weight.

## Example 2

Cloud point determination:

1. A 0.7M quaternary ammonium bromide in 2.5M Zinc Bromide water solution was prepared.
2. The solution was transferred to a jacketed flask with a stirring bar and temperature monitor.
3. The solution was warmed to 15°C above the expected cloud point. If necessary, any moisture or impurities were removed by filtration.
4. The solution was stirred, with speed adjustment to about 250 rpm, while avoiding the formation of bubbles.
5. The solution was cooled gradually (cooling rate of about 1°C/5min).
6. The sample was inspected carefully for signs of cloudiness, and the temperature at which cloudiness was observed was recorded to the nearest 0.1°C.

Note that step 3 may require the performance of an approximate cloud point measurement to roughly determine an expected cloud point. Such a measurement can be performed preparing a solution as in steps 1 and 2, and subjecting it to a temperature drop from an initial temperature which is higher than any expected cloud point, such as, for example, about 45 C.)

Examples 3-7 and 9: The cloud points and free bromine were measured as in Examples 1 and 2, respectively

## Example 3

| | Name | Structure | MW | Solubility in $ZnBr_2$ Cloudy Point | Free $Br_2$ |
|---|---|---|---|---|---|
| 2 | Triethylether Quat Bromide | 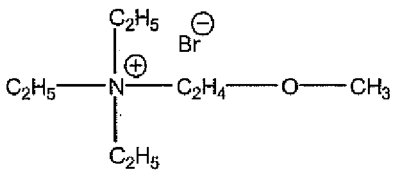 | 240 | 0.7M, <-10.5C | 0.7M 0.658% |
| 5 | Triethylpropyl Quat Bromide | 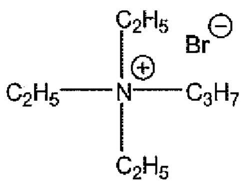 | 224 | 0.7M, 41.2C | 0.7M 0.089% |
| 6 | Mixture 50/50 mol% of 1 and 4 | | 232 | 0.7M, 10.5C | 0.7M 0.25% |

The free bromine of compound 2 at 0.7M is 0.658%, and that of compound 5 is 0.089%. Nevertheless, the 50/50 mol% ratio of the two compounds has a free bromine of 0.25% at 0.7M, which is a drop in free bromine of significantly more than 50% with respect to the free bromine of the Triethylether quat alone.

## Example 4

| | Name | Structure | MW | Solubility in $ZnBr_2$ Cloud Point | Free $Br_2$ |
|---|---|---|---|---|---|
| 1 | Dimethylbutylether Quat Bromide | 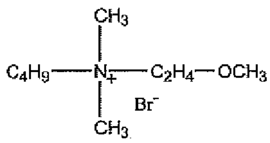 | 240 | 0.7M, 29 C | 0.7M 0.28% |
| 4 | Diethylmethylether Quat Bromide | 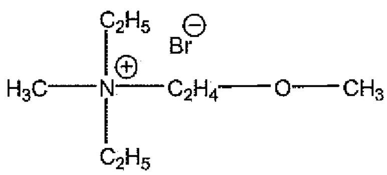 | 226 | 0.7M, <-10.5C | 0.7M 0.70% |
| 7 | Mixture 50/50 mol% of 1 and 2 | | 233 | 0.7M, 9C | 0.7M 0.22% |

Note that compound 4 differs from compound 1 only in having 1 less carbon atom on one of its methyl groups. The free bromine at 0.7M can be expected to be higher to that of compound 1.

Nevertheless, the 50/50 mol% ratio of the two compounds has a free bromine of 0.22% at 0.7M, which is similar to that of compound 1.

## Example 5

| | Name | Structure | MW | Solubility in $ZnBr_2$ Cloud Point | Free $Br_2$ |
|---|---|---|---|---|---|
| 2 | Triethylether Quat Bromide | 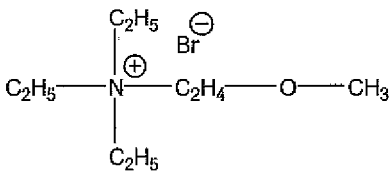 | 240 | 0.7M, <-10.5C | 0.8M 0.467% |
| 9 | Diethyl Piperidinium Bromide | 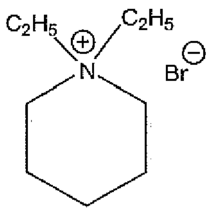 | 222 | 0.7M, 11.9C | 0.8M 0.26% |
| 12 | Mixture 50/50 mol% of 1 and 6 | | 231 | 0.7M, -3C | 0.8M 0.27% |

The free bromine of compound 2 at 0.8M is 0.467%, and that of compound 9 at 0.8M is 0.26%. A 50/50 mol% ratio of the two compounds has a surprisingly low free bromine of 0.27% at 0.8M.

## Example 6

| | Name | Structure | MW | Solubility in $ZnBr_2$ Cloud Point | Free $Br_2$ |
|---|---|---|---|---|---|
| 2 | Triethylether Quat Bromide | 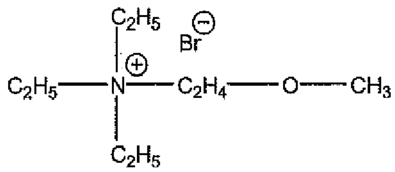 | 240 | 0.7M, <-10.5C | 0.8M 0.467% |
| 10 | EthyPropyl Piperidinium Bromide | 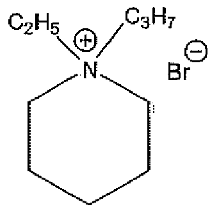 | 236 | 0.7M, 45.8C | 0.8M 0.026% |
| 13 | Mixture 50/50 mol% of 1 and 8 | | 238 | 0.7M, 18.8C | 0.8M 0.062% |

The free bromine of compound 2 at 0.8M is 0.467%, and that of compound 10 at 0.8M is 0.026%. A 50/50 mol% ratio of the two compounds has a surprisingly low free bromine of 0.062% at 0.8M.

**Example 7**

Test Conditions: 25°C
2-electrode cell set-up
Working Electrode:

Conductive graphite rod (d=0.635cm or 1/4 in, length 5cm, surface area~$10cm^2$)

Counter/Ref Electrode:

Conductive graphite rod (d=0.635cm or 1/4 inch)

Equipment: MTI battery tester
Electrolyte: 35mL 2.5m $ZnBr_2$, 0.05M $Br_2$ and 0.7M Polybromide complex
30mA for 5 hours (total ~150mAh plating capacity) non-stirred condition

Working electrodes were thoroughly cleaned by DI water rinse and dried after zinc plating. Zinc plated on working electrode was determined by measuring weight difference of working electrode before and after plating test. Zinc plating efficiencies were calculated by real zinc weight over theoretical zinc weight, which was obtained by Faraday's law of electrolysis assuming 100% conversion from 180mAh capacity.

| Complexing Agent | Plating Efficiency |
|---|---|
| MEP | 59.88% |
| Example-3 Blend | 79.05% |
| Example-4 Blend | - |
| Example-5 Blend | 74.65% |
| Example-6 Blend | 98.38% |

**Example 8**

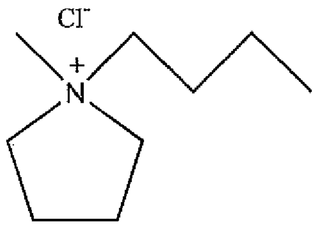

Chloroquat

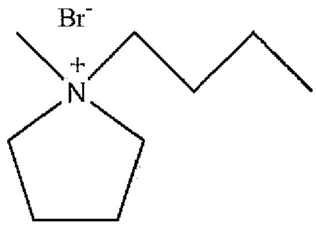

Bromoquat

Chloride ions are added to the electrolyte in amounts sufficient to reduce the amount of free bromine present and increase the electrolyte conductivity during charging of the cell. Chloride ions in the electrolyte may come from zinc chloride or quaternary ammonium chloride complexing agent.

Experiment 1 of Example 8: An aqueous electrolyte system was prepared having 0.84 M zinc bromide, 0.8 M chloroquat (N-methyl, N-butyl pyrrolidinium chloride) and 1.44 M bromine. After the sample was stirred for 24 hrs at 35°C, the amount of free bromine present in the electrolyte was 0.26%.

Experiment 2 of Example 8: An aqueous electrolyte system was prepared having 0.84 M zinc bromide, 0.8 M bromoquat (N-methyl, N-butyl pyrrolidinium bromide) and 1.44 M bromine. After the sample was stirred for 24 hrs at 35°C, the amount of free bromine present in the electrolyte was 0.25%.

| Component | Experiment 1 | Experiment 2 |
|---|---|---|
| | Concentration (M) | Concentration (M) |
| $ZnBr_2$ | 0.84 | 0.44 |
| $ZnCl_2$ | 0 | 0.40 |
| $Br_2$ | 1.44 | 1.44 |
| Bromoquat | 0 | 0.8 |
| Chloroquat | 0.8 | 0 |

**Example 9**

| | Name | Structure | MW | Solubility in $ZnBr_2$ Cloud Point | Free $Br_2$ |
|---|---|---|---|---|---|
| 14 | Dimethylethyl Gemini Quat Bromide with Diethylether Linkage | 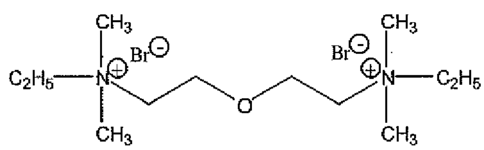 | 378 | 0.7M, 48.2C | 0.7M<br>0.71% |
| 15 | Dimethylethyl Gemini Quat Bromide with Butyl Linkage | 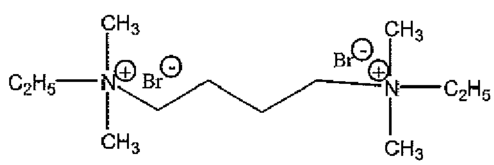 | 362 | 0.7M, 89.2C | 0.7M<br>0.59% |

The free bromine of compound 14 at 0.7 M is 0.71%, and that of compound 15 at 0.7 M is 0.59_%.

Components referred to by chemical name or formula anywhere in the specification or claims hereof, whether

referred to in the singular or plural, are identified as they exist prior to coming into contact with another substance referred to by chemical name or chemical type (*e.g.*, another component, a solvent, or *etc.*). It matters not what chemical changes, transformations and/or reactions, if any, take place in the resulting mixture or solution as such changes, transformations, and/or reactions are the natural result of bringing the specified components together under the conditions called for pursuant to this disclosure. Thus the components are identified as ingredients to be brought together in connection with performing a desired operation or in forming a desired composition. Also, even though the claims hereinafter may refer to substances, components and/or ingredients in the present tense ("comprises", "is", *etc.*), the reference is to the substance, component or ingredient as it existed at the time just before it was first contacted, blended or mixed with one or more other substances, components and/or ingredients in accordance with the present disclosure. The fact that a substance, component or ingredient may have lost its original identity through a chemical reaction or transformation during the course of contacting, blending or mixing operations, if conducted in accordance with this disclosure and with ordinary skill of a chemist, is thus of no practical concern.

The invention may comprise, consist, or consist essentially of the materials and/or procedures recited herein.

As used herein, the term "about" modifying the quantity of an ingredient in the compositions of the invention or employed in the methods of the invention refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. The term about also encompasses amounts that differ due to different equilibrium conditions for a composition resulting from a particular initial mixture. Whether or not modified by the term "about", the claims include equivalents to the quantities.

Except as may be expressly otherwise indicated, the article "a" or "an" if and as used herein is not intended to limit, and should not be construed as limiting, the description or a claim to a single element to which the article refers. Rather, the article "a" or "an" if and as used herein is intended to cover one or more such elements, unless the text expressly indicates otherwise.

Each and every patent or other publication or published document referred to in any portion of this specification is incorporated *in toto* into this disclosure by reference, as if fully set forth herein.

This invention is susceptible to considerable variation in its practice. Therefore the foregoing description is not intended to limit, and should not be construed as limiting, the invention to the particular exemplifications presented hereinabove.

## Claims

1. An electrolyte solution comprising one or more class A quat halides, each having a molecular structure selected from the group consisting of:

$$A - [(CH_2)_E - O - (CH_2)_F]_n - B$$

wherein A and B each are each independently selected from the group consisting of:

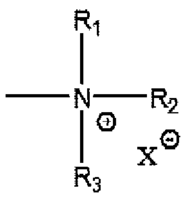

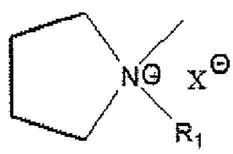

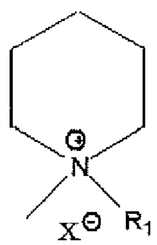

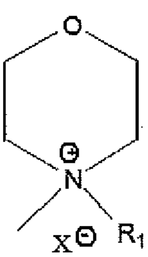

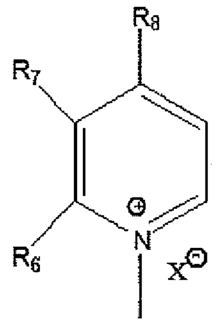

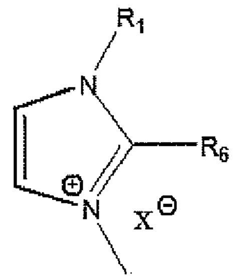

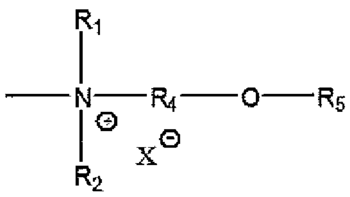

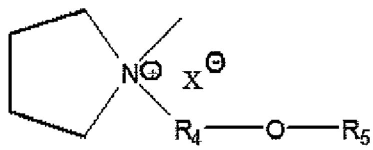

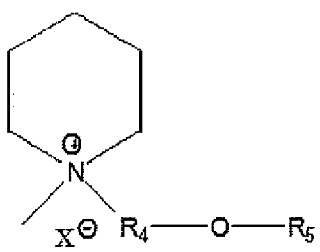

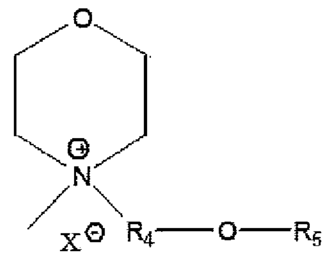

wherein

E and F are each independently in the range of 1 to 10;
n is in the range of 1 to 100;
$X^-$ is $Br^-$ or $Cl^-$, or the quat is a mixture of both bromides and chlorides;
$R_1$, $R_2$, $R_3$, are, independently, hydrogen or alkyl substituents having about 12 or fewer carbon atoms;
$R_4$ is an alkyl chain having in the range of about 1 to about 10 carbon atoms;
$R_5$ is an alkyl group having in the range of about 1 to about 6 carbon atoms; and
$R_6$, $R_7$ and $R_8$ are, independently, hydrogen or alkyl substituents having 12 or fewer carbon atoms.

**2.** An electrolyte solution as in claim 1 wherein $R_1$, $R_2$ and $R_3$ are, independently, hydrogen or alkyl substituents having in the range of about 1 and about 7 carbon atoms

**3.** An electrolyte solution as in claim 1 wherein $R_4$ is an alkyl chain having in the range of about 1 to about 4 carbon atoms.

**4.** An electrolyte solution as in claim 1 wherein $R_5$ is an alkyl group having in the range of about 1 to about 4 carbon atoms

**5.** An electrolyte solution as in claim 1 wherein the one or more class A quats is/are dimethylethyl gemini quat bromide with diethylether linkage having the following structure:

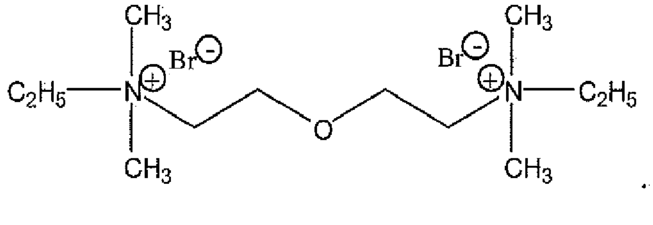

**6.** An electrolyte solution as in claim 1 further comprising one or more class B quats, each having the following structure:

$$C\text{-}(CH_2)n\text{-}D$$

wherein C and D each are each independently selected from the group consisting of:

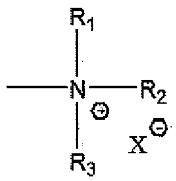

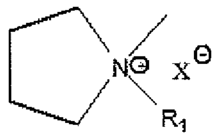

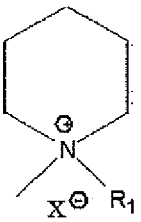

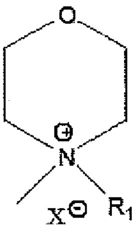

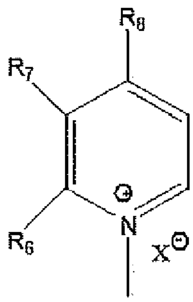

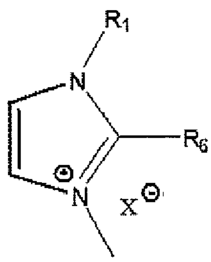

wherein

$X^-$ is Br- or Cl-, or the quat is a mixture of both bromides and chlorides;
n is in the range of 1 to 100;
$R_1$, $R_2$, $R_3$, are, independently, hydrogen or alkyl substituents having about 12 or fewer carbon atoms; and
$R_6$, $R_7$ and $R_8$ are, independently, hydrogen or alkyl substituents having 12 or fewer carbon atoms.

**7.** A zinc bromide electrolyte solution comprising one or more class A quat halides and one or more class B quat halides, each class A quat halide having a molecular structure selected from the group consisting of:

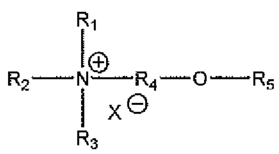

I

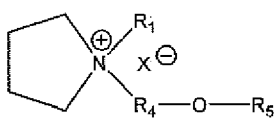

II

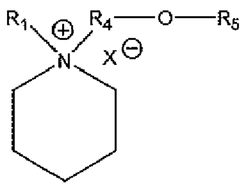

III

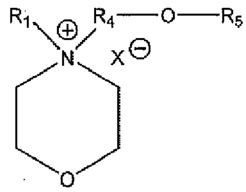

IV

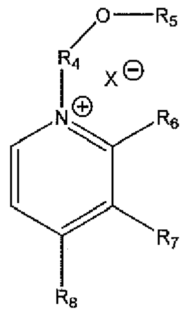

V

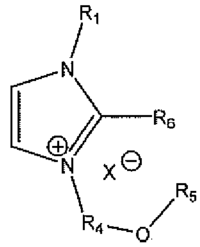

VI

and each class B quat halide having the following structure:

$$C\text{-}(CH_2)n\text{-}D$$

wherein C and D each are each independently selected from the group consisting of:

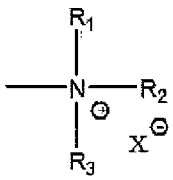

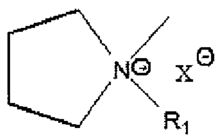

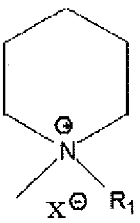

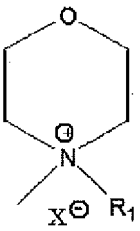

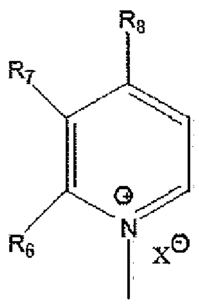

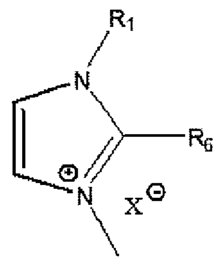

wherein

$X^-$ is $Br^-$ or $Cl^-$, or the quat is a mixture of both bromides and chlorides;
n is in the range of 1 to 100;
$R_1$, $R_2$, $R_3$, are, independently, hydrogen or alkyl substituents having about 12 or fewer carbon atoms;
$R_4$ is an alkyl chain having in the range of about 1 to about 10 carbon atoms;
$R_5$ is an alkyl group having in the range of about 1 to about 6 carbon atoms; and
$R_6$, $R_7$ and $R_8$ are, independently, hydrogen or alkyl substituents having 12 or fewer carbon atoms.

**8.** An electrolyte solution as in claim 6 wherein the one or more class B quats is/are dimethylethyl gemini quat bromide with butyl linkage having the following structure:

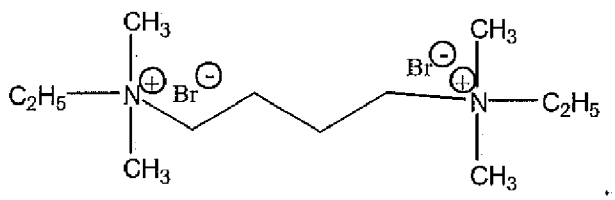

Europäisches Patentamt
European Patent Office
Office européen des brevets

# EUROPEAN SEARCH REPORT

Application Number
EP 17 15 7540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2013/003259 A1 (SASA MASAAKI [JP] ET AL) 3 January 2013 (2013-01-03)<br>* abstract *<br>* paragraphs [0002], [0011], [0030], [0038] * | 1,2<br>3-8 | INV.<br>H01M12/08<br>ADD.<br>H01M10/36 |
| X<br>A | SYLVAIN PRÉVOST ET AL: "Formation of Monodisperse Charged Vesicles in Mixtures of Cationic Gemini Surfactants and Anionic SDS",<br>LANGMUIR,<br>vol. 27, no. 2,<br>10 December 2010 (2010-12-10), pages 582-591, XP055395064,<br>US<br>ISSN: 0743-7463, DOI: 10.1021/la103976p<br>* page 583, right-hand column *<br>* page 584; table 1 *<br>* page 587, left-hand column, paragraph 2 * | 1<br>2-8 | |
| X<br>A | US 4 038 460 A (WALSH FRASER M ET AL) 26 July 1977 (1977-07-26)<br>* abstract *<br>* column 1, lines 5-9 *<br>* column 4, line 3 - column 6, line 34 *<br>* column 9 *<br>* table 3 * | 1-5<br>6-8 | TECHNICAL FIELDS SEARCHED (IPC)<br>H01M |

-/--

The present search report has been drawn up for all claims

| Place of search | Date of completion of the search | Examiner |
|---|---|---|
| The Hague | 16 November 2017 | Grave, Christian |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 15 7540

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JAMES C. NICHOL ET AL: "Bolaform Electrolytes. IV. Conductance of [alpha],[iota]-Bispyridinium Polymethylene Bromides and [beta],[beta]'-Bisquaternary Substituted Diethyl Ethers in Methanol", JOURNAL OF THE AMERICAN CHEMICAL SOCIETY, vol. 77, no. 1, 1 January 1955 (1955-01-01), pages 198-202, XP055395058, US ISSN: 0002-7863, DOI: 10.1021/ja01606a075 | 1 | |
| A | * abstract * * Experimental Part * | 2-8 | |
| A | WO 2016/028408 A1 (ALBEMARLE CORP [US]) 25 February 2016 (2016-02-25) * paragraphs [0002], [0009] - [0011] * | 1-8 | |
| A | US 4 104 447 A (WALSH FRASER M ET AL) 1 August 1978 (1978-08-01) * column 5, lines 4-18 * * column 6, lines 57-65 * | 1-8 | TECHNICAL FIELDS SEARCHED (IPC) |

The present search report has been drawn up for all claims

| Place of search | Date of completion of the search | Examiner |
|---|---|---|
| The Hague | 16 November 2017 | Grave, Christian |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number

EP 17 15 7540

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [X] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION
## SHEET B

Application Number
EP 17 15 7540

The Search Division considers that the present European patent application does not comply with the requirements of unity of invention and relates to several inventions or groups of inventions, namely:

1. claims: 2-5(completely); 1(partially)

   An electrolyte solution comprising the technical features of claim 2. Also, any claims with technical features which do not make a contribution over the prior art have been included in this group.

---

2. claims: 6-8(completely); 1(partially)

   An electrolyte solution comprising the technical features of claims 6 or 7.

---

## ANNEX TO THE EUROPEAN SEARCH REPORT ON EUROPEAN PATENT APPLICATION NO. EP 17 15 7540

This annex lists the patent family members relating to the patent documents cited in the above-mentioned European search report. The members are as contained in the European Patent Office EDP file on
The European Patent Office is in no way liable for these particulars which are merely given for the purpose of information.

16-11-2017

| Patent document cited in search report | Publication date | Patent family member(s) | Publication date |
|---|---|---|---|
| US 2013003259 A1 | 03-01-2013 | CN 102893354 A | 23-01-2013 |
| | | JP 5376052 B2 | 25-12-2013 |
| | | JP WO2011132307 A1 | 18-07-2013 |
| | | US 2013003259 A1 | 03-01-2013 |
| | | WO 2011132307 A1 | 27-10-2011 |
| US 4038460 A | 26-07-1977 | CA 1066762 A | 20-11-1979 |
| | | DE 2711694 A1 | 22-09-1977 |
| | | GB 1551873 A | 05-09-1979 |
| | | JP S52114927 A | 27-09-1977 |
| | | US 4038460 A | 26-07-1977 |
| WO 2016028408 A1 | 25-02-2016 | AU 2015303976 A1 | 09-03-2017 |
| | | CA 2958123 A1 | 25-02-2016 |
| | | CN 107078329 A | 18-08-2017 |
| | | EA 201790421 A1 | 30-06-2017 |
| | | EP 3195398 A1 | 26-07-2017 |
| | | JP 2017529660 A | 05-10-2017 |
| | | KR 20170042698 A | 19-04-2017 |
| | | PH 12017500288 A1 | 28-06-2017 |
| | | SG 11201701264Q A | 30-03-2017 |
| | | US 2017237129 A1 | 17-08-2017 |
| | | WO 2016028408 A1 | 25-02-2016 |
| US 4104447 A | 01-08-1978 | NONE | |

EPO FORM P0459

For more details about this annex : see Official Journal of the European Patent Office, No. 12/82